# NEUE EUROPÄISCHE PATENTSCHRIFT

(11) **EP 0 442 519 B2**
(45) Veröffentlichungstag und Bekanntmachung des Hinweises auf die Entscheidung über den Einspruch: **15.04.1998**
(45) Hinweis auf die Patenterteilung: 04.05.1994
(21) Anmeldenummer: 91102164.0
(22) Anmeldetag: 15.02.1991
(51) Int. Cl.: A61K 7/50, C11D 3/04

(54) **Selbstkonservierende flüssige Tensidsysteme**
Self-preserving liquid tenside systems
Systèmes tensio-actifs liquides autoconservateur

(30) Priorität: 16.02.1990 DE 4005054; 10.08.1990 DE 4025424
(43) Veröffentlichungstag der Anmeldung: 21.08.1991
(73) Patentinhaber: Joh. A. Benckiser GmbH, D-67059 Ludwigshafen (DE)
(72) Erfinder: Wäschenbach, Guido, Dr., W-6800 Mannheim 81 (DE); Baust, Henrich, W-6831 Plankstadt (DE)
(74) Vertreter: Lederer, Franz, Dr.

(56) Entgegenhaltungen:
- EP-A- 0 287 805
- EP-A- 0 323 798
- DE-A- 3 232 136
- DE-A- 3 533 601
- DE-A- 3 740 089
- JP-A- 5 929 612
- JP-A- 6 335 515
- US-A- 3 928 249
- DERWENT ABSTRACT C81-D63358
- H. JANISTYN, Taschenbuch der modernen Parfümerie und Kosmetik, 4. Aufl.age, Wissenschaftliche Verlagsgesellschaft mbH, Stuttgart, 1974, S. 728-729
- H. JANISTYN, Handbuch der Kosmetika und Riechstoffe, I. Band, Die kosmetischen Grundstoffe, Dr. Alfred Hüthig Verlag Heidelberg, 1978, S. 869, 870, 874-876
- H.P. FIEDLER, Lexikon der Hilfsstoffe für Pharmazie, Kosmetik und angrenzende Gebiete, 2. Auflage, Editio Cantor, Aulendorf, 1981, S. 869
- Kosmetikjahrbuch 1977, Verlag für chemische Industrie H. Ziolkowsky KG, Augsburg, S. 46
- E.W. FLICK, Cosmetic and Toiletry Formulations, Noyes Publications, Park Ridge, New Jersey, 1984, S. 447, 576, 577
- THE MERCK INDEX, 11th Ed., 1989, S. 5541-5542
- Firmenschrift der REWO 0283/1.1.1.7, REWOPOL SBFA 30
- Firmenschrift der REWO "REWOTERIC-Amphoteric Surfactants"

## Beschreibung

Die vorliegende Erfindung bezieht sich auf konservierungsmittelfreie, selbstkonservierende, flüssige, wasserhaltige Tensidsysteme, die einen gewissen, oft nur geringen Gehalt an einem oder mehreren Tensiden aufweisen und je nach ihrem Anwendungsgebiet unterschiedlich formuliert sind in Form von Körperreinigungsmittel wie Schaumbäder, Duschbäder, Duschgels. Haarshampoos, Gesichtswaschlotionen, Baby-Produkte, Flüssigseifen, oder, Handgeschirrspülmittel. Die Erfindung bezieht sich auf alle solche Produkte, deren Formulierung dem Fachmann grundsätzlich bekannt ist und die sich in flüssiger, wasserhaltiger, insbesondere rein wäßriger Form befinden.

Aus der Mikrobiologie ist bekannt, daß wasserhaltige tensidische Systeme, besonders wenn sie sich in den für Körperpflegeprodukten üblichen pH-Bereichen zwischen 5 und 8 bewegen, ideale Nährböden für Mikroorganismen darstellen.

Diese Mikroorganismen finden über eine Kette von Faktoren (Wasser, Rohstoffe, Anlagen, Mensch, Packstoffe, Umwelt) letztendlich Zugang zu den Final-Produkten und können infolge ihrer hohen Stoffwechselaktivität ein Produkt innerhalb kürzester Zeit verderben.

Solche Produkte müssen daher im allgemeinen Konservierungsmittel enthalten.

Wenn auch Konservierungsmittel im Einklang mit den gesetzlichen Regelungen wie Zulassung für bestimmte Produkte, Bereich. Deklarierung und Nichtüberschreitung der zulässigen Höchstmengen zum Einsatz kommen, verbleiben für Verbraucher und Hersteller immer ein Restrisiko im Bezug auf Produktsicherheit und Akzeptanz.

Beispiele hierfür sind die in jüngster Zeit in die öffentliche Kritik geratenen Stoffe wie Formaldehyd, Formaldehydabspalter, Isothiazoline, Parabene, Hexachlorophen, phenolische Substanzen allgemein, um nur einige zu nennen, wegen möglicher toxischer, cancerogener oder allergener Eigenschaften.

Ein weiterer Problempunkt ist die Einschränkung des Herstellers in seinen Export-Aktivitäten, wenn in bestimmten Ländern Zulassungsverbote für bestimmte Konservierungsstoffe existieren. (z.B. Schweden, Japan).

Aus der aufgezeigten Problematik ergibt sich, daß konservierungsmittelfreie Produkte mit eigenem, ausreichenden antimikrobiellen Potential für Verbraucher und Hersteller gleichermaßen eine Problemlösung von höchstem Interesse darstellen.

Aufgabe der vorliegenden Erfindung ist es daher flüssige, wasserhaltige Tensidsysteme zur Verfügung zu stellen, die konservierungsmittelfrei sind und selbstkonservierende Eigenschaften besitzen.

Es ist bekannt, daß hochprozentige Tenside bzw. Tensidmischungen in der Lage sind den Stoffwechsel von Mikroorganismen abzuschwächen, also eine bedingte selbstkonservierende Wirkung besitzen können. Beispiele sind Fettalkoholäthersulfat - Pasten mit einem WAS-Gehalt von 70 %.

Die Umsetzung dieser Erkenntnis in flüssige, konservierungsmittelfreie Körperreinigungsmittel scheiterte bisher an drei wesentlichen Faktoren.
i) Hohe Tensidkonzentrationen in Produkten, die direkt auf die Haut, Kopfhaut gelangen (Duschgels. Gesichtswaschlotionen, Flüssigseifen, Haarshampoos), bilden ein zu hohes Risiko einer Hautschädigung infolge Entfettung, Spannung, Rissbildung oder Erythembildung.
ii) Puffersubstanzen, die geeignet sind die Aggressivität der hohen Tensidkonzentration abzuschwächen oder möglicherweise zu kompensieren. wie z.B. Eiweißhydrolysate, bilden selbst wieder gute Nährböden für Mikroorganismen und stellen somit konservierungsmittelfreie Systeme wieder in Frage.
iii) Zusatzstoffe wie Parfümöle, Elektrolyte oder Rückfetter, die üblicherweise kosmetischen Formulierungen zugesetzt werden, z.B. Fettsäurealkylolamide, Glycerinester, Glycerin-Partialester, Fettsäureester, Fettalkohole bzw. die Äthoxylate der genannten Stoffgruppen führen in konzentrierten Tensidsystemen leicht zu Aggregatsänderungen, hin zur Pastenkonsistenz bzw. zu einer Destabilisierung des Systems (Phasentrennung, Ausflocken, Aussalzung etc.).

Es ist bekannt, Nahrungsmittel wie Fleisch oder Fisch durch Einsalzen mit Kochsalz zu konservieren. Das Salz wirkt auf osmotischem Wege austrocknend. Beim Einsalzen von Gemüse, wie Gurken, wirkt die Konservierung mittels des stark sauren Milieus aufgrund einer Milchsäuregärung. Alle diese Voraussetzungen sind bei wäßrigen Tensidsystemen nicht gegeben.

Es wurde nun überraschenderweise gefunden, daß die gegenständlichen Tensidsysteme konserviert werden können durch Einverleibung von Magnesiumchlorid in einer Menge von mindestens 2 Gew.-%, vorzugsweise 5 Gew.-% (berechnet als wasserfreies Salz). Die Obergrenze muß so gewählt werden, daß ein Aussalzen vermieden wird und liegt im allgemeinen bei 20, vorzugsweise 15 Gew.-%.

Es ist bekannt, Tensidsystemenzur Viskositätseinstellung Salze in geringen Mengen einzuverleiben.

So beschreibt die US-P 4 534 892 flüssige Detergenszusammensetzungen mit einem dispergierten, wasserunlöslichen, feinen Pulver, die neben anionischen Tensiden zur Einstellung eines hochviskosen, thixotropen Systems ein amphoteres Polymer und ein anorganisches Salz enthalten.

Die DE-OS 37 06 015 beschreibt ein flüssiges, wäßriges Reinigungsmittel für das manuelle Reinigen von Geschirr, das Alkylglucoside, Di-n-alkylsulfosuccinate und als Viskositätsregulatoren Natriumchlorid, Magnesiumchlorid oder andere Salze enthält.

Die DE-PS 29 27 278 offenbart Haarwaschmittel, die anorganische Salze zur Regelung der Viskosität enthalten können.

Die DE-OS 36 22 438 (= US-P 4 744 924) offenbart einen kosmetischen Waschrohstoff, derzur Einstellung besonderer rheologischer Eigenschaften Natrium- und Magnesium-Ionen enthalten soll.

Die DE-OS 29 36 934 offenbart ein Mittel zur Behandlung von Fasermaterialien, z.B. Haaren, welches Polymere, ein nichtionisches, oberflächenaktives Mittel und mindestens ein Alkalimetallsalz enthalten soll.

Die GB-P 1 217 322 offenbart Reinigungsmittel insbesondere für die Körperpflege, bei denen die Aktivsubstanz aus bestimmten Sulfobernsteinsäureestern besteht und die auch in gewissen Fällen anorganische Salze enthalten können, obwohl eine größere Menge solcher Elektrolyte nicht gewünscht ist und am besten vermieden wird.

Die EP-A-287 805 offenbart die Verwendung einer Kombination aus a) einem Salz der Ameisensaure, b) Benzoesäure oder einem Salz der Benzoesäure und c) einer anorganischen oder aliphatischen organischen Säure zur Konservierung von Haar- und Körperreinigungsmitteln.

Aus keiner dieser Druckschriften geht hervor, daß anorganische Salze in solchen Tensidsystemen konservierend wirken können und ihre Verwendung herkömmliche Konservierungsmittel ersetzen kann.

Die Tensidsysteme der vorliegenden Erfindung enthalten im allgemeinen 2 bis 50 Gew.-%, vorzugsweise 5 bis 35 Gew.-% eines oder mehrerer Tenside.

Gegenstand der Erfindung ist somit die Verwendung von Magnesiumchlorid in einer Menge von mindestens 2 Gew.-%. vorzugsweise mindestens 5 Gew.-% (berechnet als wasserfreies Salz), zur Konservierung konservierungsmittelfreier, flüssiger, wasserhaltiger Tensidsysteme der genannten Art enthaltend 2-50 Gew.-%, vorzugsweise 5-35 Gew.-% eines oder mehrerer Tenside.

Ein besonders bevorzugtes Tensidsystem enthält 2-15, insbesondere 5-10 Gew.-% Magnesiumchlorid (berechnet als wasserfreies Magnesiumchlorid). bzw. 4-30 Gew.-%, vorzugsweise 10-25 Gew.-% MgCl₂ (berechnet als MgCl₂.6H₂O).

Geeignete Tenside sind

### Fettsäuremethyltauride

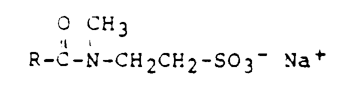

- R =: Cocos-, Stearinsäure, Ölsäure
- z.B.: Hostapone CT, STT, TF (Hoechst)

### Fettsäuretauride

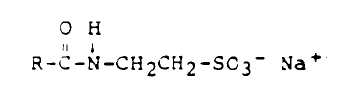

- R =: Cocosfettsäure
- z.B.: Hostapon KTW (Hoechst)

### Isäthionate

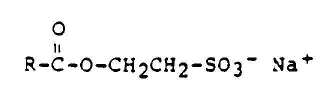

- R =: Cocosfettsäure
- z.B.: Hostapon KA (Hoechst)

### Sarkoside

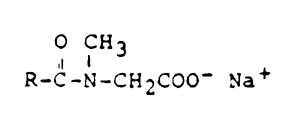

- R =: Cocosfettsäure, Laurinsäure
- z.B.: Medialan KF (Hoechst)
Medialan CD (Hoechst)

### Eiweißfettsäure-Kondensationsprodukte

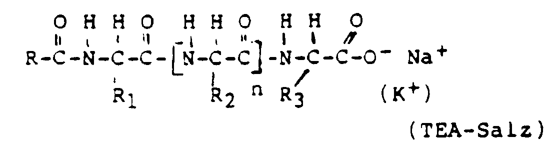

Kation: Na, K, TEA
- R =: Cocosfettsäure
- n =: 0 bis 32
- z.B.: Lamepon S (Henkel)
Lamepon S2 (Henkel)
Lamepon S-TR (Henkel)
Geliderm 3000 (Deutsche Gelatine Fabriken Stoess & Co)

### N-Cocoylglutaminsäure-mono-Na-Salz

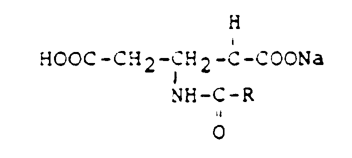

- z.B.: N-Cocoylglutaminsäure-mono-Na-Salz (Hoechst)

### Äthercarbonsäuren

R-O(CH₂CH₂O)ₙ-CH₂COONa

- R =: Cocos- oder Lauryl bzw. C₁₂-C₁₆
- n =: 2,5 bis 18
- z.B.: Marlinale CM (Hüls)
Akypo RLM (Chem-y)

### Fettalkoholäthersulfate

Erweiterung Ethoxilierungsgrad:

R-O(CH₂CH₂O)ₙSO₃⁻Na

- n =: 2 bis 18

### Cocoamphoacetate

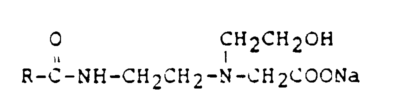

- z.B.: Rewoteric AM-2C/NM (Rewo)
Rexoteric XCO-40 (Rexolin)
Miranol CM conc.N.P. (Miranol)
Dehyton G (Henkel)

### Cocoamphodiacetate

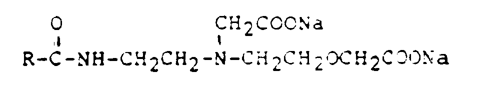

- z.B.: Rewoteric AM-2C (Rewo)
Miranol C2M conc.N.P. (Miranol)

### Alkylpolyglucoside

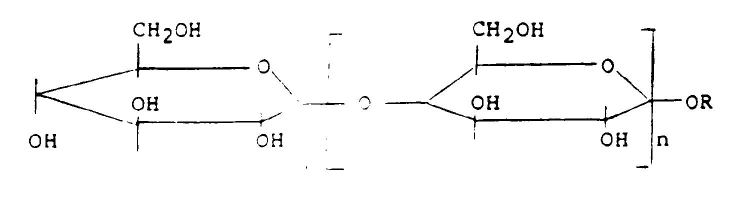

- n =: vorzugsweise 0 bis 4
- R =: C₈-C₁₂ Alkyl-
- z.B.: Triton CG 110 (Rohm & Haas)
Lutensol GD 70 (BASF)
Ferner:

### Saccharoseester

- z.B.: Grillotene (Grillo-Werke)

### Sorbitanfettsäureester-Äthoxylate

- z.B.: Tween-Reihe (Atlas-Chemie)

### Aminoxide

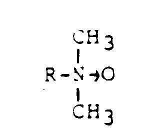

- R =: C₁₂-C₁₈
- z.B.: Empigen OB (Albright & Wilson)
Aminoxid WS35 (Th. Godschmidt)

Eine besonders geeignete, überraschenderweise bereits für sich nicht infizierbare Tensidkombination, die im Rahmen der vorliegenden Erfindung gefunden wurde, besteht aus
a) 20-90 Gew.-%, vorzugsweise 67-75 Gew.-% eines oder mehrerer Sulfobernsteinsäureester insbesondere des nachstehend dargestellten Typs A;
b) 9-79, vorzugsweise 9-49 Gew.-%, insbesondere 22-30 Gew.-% einer oder mehrerer Verbindungen aus der Gruppe der anionischen Tenside.
c) 1-10 Gew.-%, vorzugsweise 2-5 Gew.-% einer oder mehrerer amphoterer Verbindungen.
d) 0-5 Gew.-%, vorzugsweise 1-2 Gew.-% einer oder mehrerer nichtionischen Tenside.

In einer besonderen Ausführungsform betrifft die Erfindung daher ein konservierungsmittelfreies, selbst konservierendes, flüssiges wasserhaltiges Tensidsystem in Form eines Körperreinigungsmittels oder Handgeschirrspülmittels enthaltend 2-50 Gew.-%, vorzugsweise 5-35 Gew.-% einer Tensidkombination, dadurch gekennzeichnet, daß die Tensidkombination aus
a) 20-90 Gew.-%, vorzugsweise 67-75 Gew.-% eines Sulfobernsteinsäure-Halbesters der allgemeinen Formel 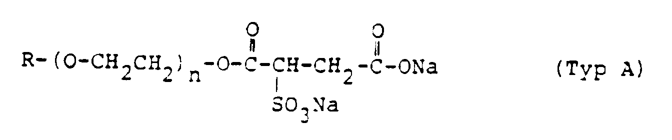 worin R C₇-C₁₇-Alkyl oder Rizinol-Alkyl und n = 2 - 4 bedeuten.
b) 9-79, vorzugsweise 9-49 Gew.-%, insbesondere 22-30 Gew.-% einer oder mehrerer Verbindungen aus der Gruppe der anionischen Tenside.
c) 1-10 Gew.-%, vorzugsweise 2-5 Gew.-% einer oder mehrerer amphoterer Verbindungen
d) 0-5 Gew.-%, vorzugsweise 1-2 Gew.-% einer oder mehrerer nichtionischen Tenside besteht und weiterhin dadurch gekennzeichnet, daß das System 2-25 Gew.-%, vorzugsweise 5-15 Gew.-% (berechnet als wasserfreies Salz) Magnesiumchlorid enthält.

Geeignete Sulfobernsteinsäureester sind insbesondere Sulfobernsteinsäure-Halbester der allgemeinen Formeln 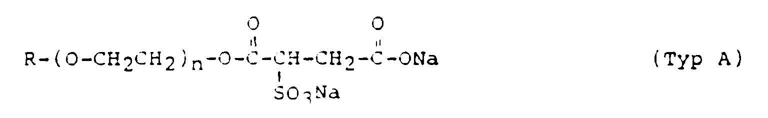 worin R C₇-C₁₇-Alkyl oder Rizinol-Alkyl und n = 2 - 4 bedeuten.

Diese können auch in Kombination mit Derivaten der allgemeinen Formel 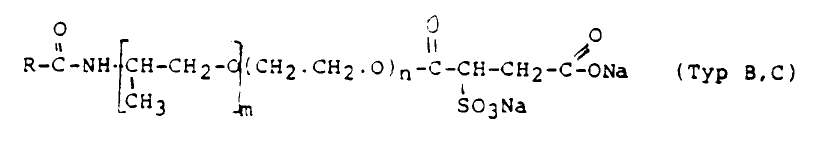 worin R und n obige Bedeutung besitzen und m = 1 oder 0 ist, eingesetzt werden.

Bevorzugte Sulfobernsteinsäure-Halbester sind solche des Typs A. Beispiele des Typs B sind solche bei denen in der allgemeinen Formel R = C₇-C₁₇-Alkyl und m = 1 ist, 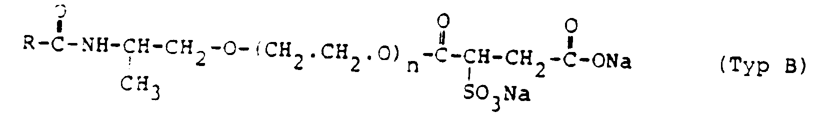 des Typs C solche, bei denen in der allgemeinen Formel
- R =: Rizinol-Alkyl, m = 0 und n = 1 ist

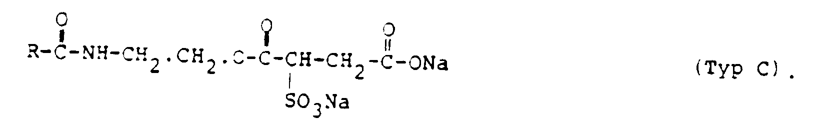

Wenn Kombinationen verwendet werden, werden die Sulfobernsteinsäure-Halbester des Typs A, B und C vorzugsweise in einem Verhältnis von A:B:C = (2-15):(1-2):1, vorzugsweise (2-10):1:1 eingesetzt.

Das anionische Tensid liegt in der Tensidkombination in einer Menge von 9-79 Gew.-%, vorzugsweise 22-30 Gew.-%(bezogen auf die Tensidkombination) vor. Geeignete anionische Tenside sind Alkylsulfate, Alkyläthersulfate, sek. Alkansulfonate und Olefinsulfonate.

Insbesondere eignen sich
Fettalkoholsulfate der allgemeinen Formel

R-O-SO⁻₃X

wobei R C₁₂-C₁₆-Alkyl und X ein Alkalimetall-, Erdalkalimetall- oder Alkanolammonium-Ion bedeuten; Fettalkoholäthersulfate der allgemeinen Formel

R-(-O-CH₂CH₂)ₙ-O-SO⁻₃X

wobei R C₇-C₁₇-Alkyl, n eine Zahl von 2 - 10 und X ein Alkalimetall-, Erdalkalimetall- oder ein Alkanolammonium-Ion bedeuten;
sekundäre Alkansulfonate der allgemeinen Formel 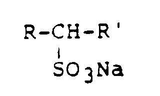 wobei R und R' überwiegend C₁₂-C₁₆-Alkyl bedeutet;
α-Olefinsulfonate der allgemeinen Formel

R-CH=CH-CH₂CH₂-SO₃Na

wobei R C₁₂-C₂₀ Alkyl oder Alkenyl bedeutet.

Die amphotere Verbindung liegt in der Tensidkombination in einer Menge von 1 - 10 Gew.-%, vorzugsweise 2 - 5 Gew.-% (bezogen auf die Tensidkombination) vor.

Als amphotere Verbindungen eignen sich Betaine, insbesondere Amido-Alkylbetaine der allgemeinen Formel 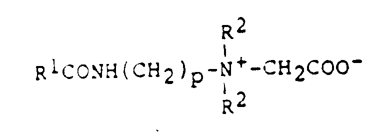 und Alkylbetaine der allgemeinen Formel 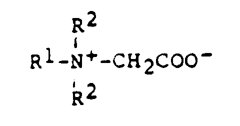 wobei in den allgemeinen Formeln R¹ C₇-C₁₇-Alkyl, R² C₁-C₄-Alkyl und p = 2 oder 3 bedeuten.

Das nichtionische Tensid muß nicht zwingend vorhanden sein. Es kann in einer Menge von 0 - 5 Gew.-%, vorzugsweise 1 - 2 Gew.-% (bezogen auf die Tensidkombination) vorliegen.

Als nichtionische Tenside eignen sich Fettsäureglycerinpolyethylenglycolester, Fettsäurepolyethylenglycolester und Glycerinethoxylate.

Die stabilen, flüssigen erfindungsgemäßen Tensidsysteme sind klar bis emulsionsartig und sind in dem Viskositätsbereich von wasserdünn bis Flüssig-Gel einstellbar.

Die erfindungsgemäßen Tensidsysteme können neben dem Tensid und dem Salz noch andere übliche Zusätze enthalten. So kann ein Zusatz einer geringen Menge 1,2-Propylenglycol und Parfümöl je nach Verwendungszweck vorteilhaft sein.

Die erfindungemäßen Tensidsysteme als Körperreinigungsmittel weisen im allgemeinen einen pH-Wert von 5 - 8, vorzugsweise von 5,5 - 7 auf.

Ein besonders bevorzugtes Körperreinigungsmittel gemäß vorliegender Erfindung in Form eines Duschgels besteht aus
12 - 30 Gew.-% einer Tensidkombination aus
58 - 62 Gew.-% Di-Na-Laurylalkoholpolyglycolethersulfosuccinat,
6 - 8 Gew.-% Di-Na-Cocosfettsäureisopropanolamidpolyglycolethersulfosuccinat,
5 - 7 Gew.-% Di-Na-Rizinolsäuremonoethanolamidsulfosuccinat,
22 - 25 Gew.-% Na-Laurylethersulfat (2-3 Mol EO),
2 - 4 Gew.-% Cocosfettsäure-amidopropylbetain,
0,5 - 1,5 Gew.-% Glycerin-Polyethylenglycoloxystearat,
0,5 - 2,0 Gew.-% 1,2-Propylenglycol.
0,4 - 1,5 Gew.-% Parfümöl,
und
2 - 14 Gew.-% Magnesiumchlorid (wasserfrei)
Rest Wasser.

### Beispiel 1

Eine erfindungsgemäße Tensidmischung I wurde mit einer konventionellen Tensidmischung II mit und ohne Elektrolytzusatz (Magnesiumchlorid) bei Anwendung in einem Duschgel verglichen.

| Tensidmischung I (Erfindung) | |
|---|---|
| Rohstoffe * | Gew.-% |
| A) Di-Na-Laurylalkoholpolyglycolethersulfosuccinat | 60,20 |
| B) Di-Na-Cocosfettsäureisopropanolamidpolyglycolethersulfosuccinat | 7,00 |
| C) Di-Na-Rizinolsäuremonoethanolamidsulfosuccinat | 5,80 |
| D) Na-Laurylethersulfat, 2-3 Mol EO | 23,10 |
| E) Cocosfettsäure-amidopropylbetain | 2,90 |
| F) Glycerin-Polyethylenglycoloxystearat | 1,00 |
| | 100,00 |

| | |
|---|---|
| * alle verwendeten Rohstoffe enthalten kein Konservierungsmittel | |

| Tensidmischung II (konventionelles System) | |
|---|---|
| Rohstoffe * | Gew.-% |
| Na-Laurylethersulfat, 2-3 Mol EO | 89,00 |
| Cocosfettsäure-amidopropylbetain | 10,00 |
| Glycerin-Polyethylenglycoloxystearat | 1,00 |
| Summe Tensidmischung II | 100,00 |

| | |
|---|---|
| * alle verwendeten Rohstoffe enthalten kein Konservierungsmittel | |

Die erfindungsgemäße Tensidmischung I wurde in verschiedener Menge in Duschgelen unterschiedlicher Zusammensetzung geprüft und verglichen mit einer konventionellen Tensidmischung II (B-Standard II) und einem mit Dibrom-dicyanobutan konservierten Duschgel (A-Standard I).

Die Zusammensetzung der Duschgele kann der nachstehenden Tabelle I entnommen werden.

**Tabelle I**

| | Verauchsbezeichnungen: | | | | | |
|---|---|---|---|---|---|---|
| Rohstoffe | A (Standard I) % | B (Standard II) % | C % | F % | H % | I % |
| Tensid - Mischung I | | - | 12,5 | 12,5 | 30,0 | 40,0 |
| Tensid - Mischung II | | 12,5 | - | - | - | - |
| 1,2 - Propylenglycol | | - | 0,63 | 0,63 | 1,5 | 2,0 |
| Parfumöl | | 0,41 | 0,41 | 0,41 | 1,0 | 1,3 |
| Mg - Chlorid | | - | - | 12,0 | - | - |
| Wasser, keimfrei | | ad 100 | ad 100 | ad 100 | ad 100 | ad 100 |
| Summe % | | 100,00 | 100,00 | 100,00 | 100,00 | 100,00 |
| pH conc. (Einstellung mit Na OH bzw. HCl) | | 6,1 | 6,1 | 6,1 | 6,1 | 6,1 |
| Tensid -Gehalt % | | 12,5 | 12,5 | 12,5 | 30,0 | 40,0 |

Im Konservierungsbelastungstest nach Wallhäuser und nach FIP (Fresenius) wurden die Körperreinigungsmittel auf ihre "ausreichende Konservierung" untersucht.

Der Begriff "ausreichende Konservierung" wird in Wallhäuser "Desinfektion-Konservierung", 4. Auflage 1988. Georg Thieme Verlag Stuttgart auf den Seiten 408 - 415 definiert.

Die zu untersuchenden Produkte werden dabei mit folgenden Mikroorganismen (M.O.) mit einer Belastung von 10⁵ Keimen/ml infiziert:

| Bezeichnung M.O. | Gruppierung M.O. |
|---|---|
| Staphlococcus aureus = | grampositive Bakterien |
| Pseudomonas aeruginosa = | gramnegative Bakterien |
| Escherichia coli = | gramnegative Bakterien |
| Candida albicans = | Hefen |
| Aspergillus niger = | Schimmelpilze |

Eine ausreichende Konservierung liegt vor, wenn nach Einbringung der Testkeime die Bakterien
- in weniger als 3 Wochen auf weniger als 10² Keime/ml (Wallhäuser)
- nach 14 Tagen auf mindestens 10² Keime/ml (FIP) zurückgehen und die Hefen und Pilze
- in weniger als 3 Wochen auf weniger als 10³ Keime/ml (Wallhäuser)
- nach 14 Tagen auf mindestens 10⁴ Keime/ml zurückgehen und nach 28 Tagen keine Vermehrung eintritt (FIP)

### Ergebnisse

### Konservierungsbelastungstest nach Wallhäuser (Einzelstämme)

Die elektrolythaltige Rezeptur F, bewirkt bereits nach 14 Tagen eine Keimreduktion für Hefen und Bakterien auf Null, die auch nach 28 Tagen unverändert anhält. Eine gewisse fungizide Wirkung der elektrolytfreien Rezeptur C war nicht reproduzierbar.

### Konservierungsbelastungstest - FIP Methoden (Mischkulturen)

Hier wurden die Testbedingungen insofern verschärft, als zusätzlich mit dem als sehr resistent geltenden Bakterienstamm Bacillus subtilis gearbeitet wurde (Sporenbildner). Ergänzend zur Wallhäuseruntersuchung wurde ein mit Dibromdicyanobutan und Phenoxyethanol konserviertes Duschgel A und ein nicht konserviertes, marktüblich aufgebautes Duschgel B als Standard mitgetestet.

Während die erfindungsgemäße Formulierung F, hinsichtlich der Bakterien-Reduktion auf annähernd gleichen, teilweise sogar auf besserem Keimreduktions-Niveau lag wie die konservierte Formel A, zeigte die unkonservierte, konventionell aufgebaute Formel B bereits nach 7 Tagen erwartungsgemäß einen Anstieg Keimgehaltes auf ca. 10⁶ Keime/ml, der sich nach 14, 21 und 28 Tagen auf diesem hohen Niveau stabilisierte.

Auch hinsichtlich der Abtötung von Hefen und Pilzen ist eine hohe Wirksamkeit gegeben.

Zusammengefaßt wird festgestellt, daß die erfindungsgemäßen Formulierungen die Voraussetzungen für eine ausreichende Konservierung gemäß den Mindestforderungen von Wallhäuser (Kosmetika) als auch denen von FIP (Dermatika) bei weitem erfüllen.

### Beispiel 2

Ein erfindungsgemäßes Tensidsystem wurde wie folgt formuliert:

| | % |
|---|---|
| Leitungswasser | 71,47 |
| Genapol LRO 70 (Fettalkoholäthersulfat, Na-Salz C₁₂₋₁₄ 70:30, 3 Mol EO 70 %ig, unkonserviert) | 17,86 |
| MgCl₂x6H₂O | 10,67 |
| pH (eingestellt mit 1nHCl) | 6,50 |
| Summe | 100,00 |
| Mg-Salz (wasserfrei) | 5,00 |
| Tensidgehalt | 12,50 |

Das Produkt ist ein blankes, farbloses Gel.

Das Produkt wurde einem Konservierungsbelastungstest nach Wallhäuser unterzogen.

| Teststämme | Versuchsbeginn | 1 Tag | 1 Woche |
|---|---|---|---|
| Pseudomonas aeruginosa | 9,6 x 10⁴/ml | 260 | 0 |
| Candida albicans | 1,6 x 10³/ml | 0 | 0 |

### Beispiele 3 und 4

Folgende Tensidsysteme wurden formuliert und getestet:

| Komponente | Menge % | |
|---|---|---|
| | Beispiel 3 | Beispiel 4 |
| Leitungswasser | 66,66 | 56,20 |
| Genapol LRO 70 | 12,87 | 10,90 |
| Setacin 103 spez | 6,19 | - |
| Rewoder S1333 | 0,59 | - |
| Rewopol SBZ | 0,59 | - |
| Tego Betain L7 | 1,20 | 4,84 |
| Marlinat CM100 | - | 2,94 |
| NaOH, 4 %ig | - | 4,48 |
| Cetiol HE | - | 0,35 |
| MgCl₂x6H₂O | 10,67 | 19,06 |
| 1,2 Propylenglykol | 0,63 | 0,63 |
| Cremophor RM410 | 0,13 | 0,13 |
| Parfüm | 0,41 | 0,41 |
| pH Einstellung aus | 6,50 | 6,50 |
| Summe | 100,00 | 100,00 |
| Mg-Salz (wasserfrei) | 5,00 | 8,93 |
| Tensidgehalt | 12,50 | 12,50 |

- Genapol LRO 70: = Fettalkoholäthersulfat, Na-Salz, C₁₂₋₁₄ 70:30; 3 Mol EO (70 %ige Ware)
- Setacin 103 spez. (40 %ig): = Sulfobernsteinsäure-Ester, Typ A
- Rewoderm S1333 (40 %ig): = Sulfobernsteinsäure-Ester, Typ C
- Rewopol SBZ (49 %ig): = Sulfobernsteinsäure-Ester, Typ B
- Teo Betain L7 (30 %ig): = Alkylamidopropylbetain
- Marlinat CM 100 (91 %ig): = Fettalkoholpolyglykolether-Carbonsäure C12/14 70:30; 10 Mol EO
- Cetiol HE (100 %ig): = Ethoxylierter Glycerinester

Das Produkt des Beispiels 3 ist blank und viskos, das Produkt des Beispiels 4 ist blank und weist eine Viskosität von 706 mPas auf.

Die Produkte wurden einem Konservierungbelastungstest nach Wallhäuser unterzogen.

| Teststämme | Versuchsbeginn Beispiel: | | 1 Tag Beisp. | | 1 Woche Beisp. | |
|---|---|---|---|---|---|---|
| | 3 | 4 | 3 | 4 | 3 | 4 |
| Pseudomonas aeruginosa | 9,6x10⁴ | 9,6x10⁴ | 0 | 0 | 0 | 0 |
| Candida albicans | 1,6x10³ | 1,6x10³ | 0 | 0 | 0 | 0 |

In den folgenden Beispielen werden folgende Tensidbezeichnungen verwendet:
- NLES: = Na-Laurylethersulfat
- NLS: = Na - Laurylsulfat
- SAS: = Sekundäres Alkansulfonat
- AOS: = α - Olefinsulfonat
- LAS: = Lineares Alkylbenzolsulfonat
- AAB: = Alkylamidopropyl - Betain
- SBE Typ A: = Sulfobernsteinsäure Halbester Typ A
- SBE Typ B: = Sulfobernsteinsäure Halbester Typ B
- SBE Typ C: = Sulfobernsteinsäure Halbester Typ C

### Beispiel 5 (Vergleich)

Eine 12,5 %-ige wäßrige Tensidlösung (pH 6.0 - 6.5) wurde ohne weitere Zusätze mikrobiologisch unter Verwendung von Mischkulturen untersucht.

| | SBE | NLES |
|---|---|---|
| Ausgangskeimzahl (Belastung) | Bakterien 10⁵ bis 10⁶ | Bakterien 10⁵ bis 10⁶ |
| Keimzahl : Keime / ml | | |
| nach 7 Tagen | 10⁴ | 10⁶ |
| nach 14 Tagen | 10⁵ | 10⁵ |
| nach 28 Tagen | 10⁵ | 10⁶ |

Eine geringfügige bakteriostatische Wirkung von SBE ist feststellbar im Gegensatz zu NLES, welches praktisch wirkungslos ist.

### Beispiel 6 (Vergleich)

Ein SBE-Monosystem wurde mit einer SBE-Kombination in 12,5% wäßriger Lösung (pH 6,0 - 6.5) ohne weitere Zusätze verglichen

| TENSIDE | SBE - Monosystem % | SBE - Kombination % |
|---|---|---|
| SBE Typ A (100 %ig) | 100,0 | 82,5 |
| SBE Typ B | 0 | 7,9 |
| SBE Typ C | 0 | 9,6 |
| Summe | 100,0 | 100,0 |
| TENSID (E) % | 12,5 | 12,5 |
| WASSER % | ad 100,0 | ad 100,0 |

### Mikrobiologische Ergebnisse,

### Beimpfung mit Mischkulturen:

| | SBE - Monosystem | | SBE -Kombinationen | |
|---|---|---|---|---|
| Ausgangskeimzahl (Befastung) | Bakterien 10⁵ bis 10⁶ | Hefen 10⁵ bis 10⁶ | Bakterien 10⁵ bis 10⁶ | Hefen 10⁵ bis 10⁶ |
| Keimzahl: Keime / ml | | | | |
| nach 7 Tagen | 10⁴ | 10⁵ | 40 | 10³ |
| nach 14 Tagen | 10⁵ | 10⁵ | 0̸ | 10⁵ |
| nach 28 Tagen | 10⁵ | 10⁵ | 0̸ | 10⁴ |

Die Ergebnisse zeigen eine mäßige bakterizide, jedoch nur geringe fungizide Wirksamkeit der Kombination.

### Beispiel 7 (Vergleich)

Eine SBE-Kombination mit verschiedenen weiteren Tensiden wurde in 12.5 %-iger wäßriger Lösung ohne weitere Zusätze mikrobiologisch mit Mischkulturen getestet:

| TENSIDE | E % |
|---|---|
| SBE Typ A | 60,2 |
| SBE Typ B | 7,0 |
| SBE Typ C | 5,8 |
| Tenside a, b, c, d, e * | 23,1 |
| Glycerin-Polyethylenglycoloxostearat | 1,0 |
| AAB | 2,9 |
| Summe | 100,0 |
| E TENSIDE % | 12,5 |
| WASSER % | ad 100,0 |

### Mikrobiologische Ergebnisse,

### Beimpfung mit Mischkulturen:

| Ausgangskeimzahl (Belastung) | Bakterien | | | | | Hefen + Pilze | | | | |
|---|---|---|---|---|---|---|---|---|---|---|
| | a | b | c | d | e | a | b | c | d | e |
| | 10⁵ bis 10⁶ | | | | | 10⁵ bis 10⁶ | | | | |
| Keimzahl : Keime / ml | | | | | | | | | | |
| nach 7 Tagen | 10⁶ | 10⁵ | 10⁴ | 10⁶ | 10⁶ | 10³ | 0̸ | 10² | 10³ | 10³ |
| nach 14 Tagen | 10⁵ | 10⁵ | 10⁴ | 10⁶ | 10⁶ | 0̸ | 0̸ | 0̸ | 10³ | 10³ |
| nach 28 Tagen | 10⁶ | 10⁶ | 10⁵ | 10³ | 10⁶ | 0̸ | 0̸ | 0̸ | 0̸ | 0̸ |

| | | | | | | | | | | |
|---|---|---|---|---|---|---|---|---|---|---|
| * a = NLES b = NLS c = SAS d = AOS e = LAS | | | | | | | | | | |

Eine geringe fungistatische Wirkung kann beobachtet werden.

### Beispiel 8

Dieses Beispiel zeigt,wie durch den erfindungsgemäßen Einsatz von 19.0 Gew.-% MgCl₂.6aq ein mikrobiologisch anfälliges Tensidsystem (pH 6.0 - 6.5) in ein stark bakterizides und fungizides System überführt werden kann.

| TENSIDE | ohne Mg - Salze % | mit Mg - Salzen % |
|---|---|---|
| SBE Typ A | 60,2 | 60,2 |
| SBE Typ B | 7,0 | 7,0 |
| SBE Typ C | 5,8 | 5,8 |
| NLES | 23,1 | 23,1 |
| AAB | 2,9 | 2,9 |
| Glycerin-Polyethylenglycoloxostearat | 1,0 | 1,0 |
| Summe | 100,0 | 100,0 |
| TENSIDE % | 12,5 | 12,5 |
| Mg Cl2 x 6 aq | 0 | 19,0 |
| WASSER % | ad 100,0 | ad 100,0 |

### Mikrobiologische Ergebnisse,

### Beimpfung mit Mischkulturen:

| | ohne Mg - Salze | | mit Mg - Salzen | |
|---|---|---|---|---|
| Ausgangskeimzahl (Belastung) | Bakterien 10⁵ bis 10⁶ | Hefen + Pilze 10⁵ bis 10⁶ | Bakterien 10⁵ bis 10⁶ | Hefen + Pilze 10⁵ bis 10⁶ |
| Keimzahl : Keime / ml | | | | |
| nach 7 Tagen | 10⁶ | 10³ | 0̸ | 10³ |
| nach 14 Tagen | 10⁵ | 0̸ | 0̸ | 10¹ |
| nach 28 Tagen | 10⁶ | 0̸ | 0̸ | 0̸ |

### Beispiel 9

Zeigt die bakterizide Wirkung von Magnesiumchlorid in einem Tensidsystem, in dem nur ein Sulfobernsteinsäureester Typ A mit anderen Tensiden kombiniert ist (pH 6,0 - 6,5).

| TENSIDE | ohne Mg - Salze % | mit Mg - Salzen % |
|---|---|---|
| SBE Typ A | 73,0 | 73,0 |
| NLES | 23,1 | 23,1 |
| AAB | 2,9 | 2,9 |
| Glycerin - PEG - oxostearat | 1,0 | 1,0 |
| Summe | 100,0 | 100,0 |
| TENSIDE % | 12,5 | 12,5 |
| Mg Cl₂x 6aq % | 0 | 19,0 |
| WASSER % | ad 100,0 | ad 100,0 |

### Mikrobiologische Ergebnisse,

### Beimpfung mit Mischkulturen:

| | ohne Mg - Salze | | mit Mg - Salzen | |
|---|---|---|---|---|
| Ausgangskeimzahl (Belastung) | Bakterien 10⁵ bis 10⁶ | Hefen + Pilze 10⁵ bis 10⁶ | Bakterien 10⁵ bis 10⁶ | Hefen + Pilze 10⁵ bis 10⁶ |
| Keimzahl : Keime / ml | | | | |
| nach 7 Tagen | 10⁵ | 10⁴ | 0̸ | 10³ |
| nach 14 Tagen | 10⁴ | 10⁴ | 0̸ | 0̸ |
| nach 28 Tagen | 10⁵ | 10⁶ | 0̸ | 0̸ |

### Beispiel 10

Zeigt unterschiedliche Mischungsverhältnisse der Tensidkombination (pH 6,0 - 6,5)

| TENSIDE | SBE : NLES = 75 : 25 % | SBE : NLES = 25 : 75 % |
|---|---|---|
| SBE Typ A | 60,2 | 19,8 |
| SBE Typ B | 7,0 | 1,9 |
| SBE Typ C | 5,8 | 2,3 |
| NLES | 23,1 | 72,1 |
| AAB | 2,9 | 2,9 |
| Glycerin - PEG - oxostearat | 1,0 | 1,0 |
| Summe | 100,0 | 100,0 |
| E TENSIDE % | 12,5 | 12,5 |
| Mg Cl₂x 6aq % | 19,0 | 19,0 |
| WASSER % | ad 100,0 | ad 100,0 |

### Mikrobiologische Ergebnisse,

### Beimpfung mit Mischkulturen:

| | SBE : NLES = 75 : 25 | | SBE : NLES = 25 : 75 | |
|---|---|---|---|---|
| Ausgangskeimzahl (Belastung) | Bakterien 10⁵ bis 10⁶ | Hefen + Pilze 10⁵ bis 10⁶ | Bakterien 10⁵ bis 10⁶ | Hefen + Pilze 10⁵ bis 10⁶ |
| Keimzahl : Keime / ml | | | | |
| nach 7 Tagen | 0̸ | 10³ | 0̸ | 0̸ |
| nach 14 Tagen | 0̸ | 10¹ | 0̸ | 0̸ |
| nach 28 Tagen | 0̸ | 0̸ | 0̸ | 0̸ |

### Beispiele 11

Eine 12,5 %-ige wäßrige NLES-Tensidlösung mit unterschiedlichen Mengen MgCl₂.6aq wurde mikrobiologisch mit Mischkulturen untersucht. Während bei einem Zusatz von 2,1 % noch keine Wirkung eintritt, ist bei einem Zusatz von 6,4 % eine leichte Wirkung und bei 10,7 % eine deutliche Wirkung erkennbar

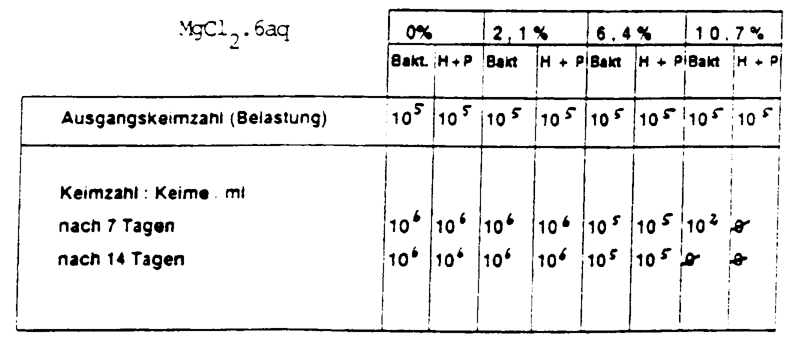

## Patentansprüche

1. Verwendung von Magnesiumchlorid in einer Menge von mindestens 2 Gew.%, vorzugsweise mindestens 5 Gew.% (berechnet als wasserfreies Salz), zur Konservierung konservierungsmittelfreier, flüssiger, wasserhaltiger Tensidsysteme in Form eines Körperreinigungsmittels oder Handgeschirrspülmittels enthaltend 2-50 Gew.%, vorzugsweise 5-35 Gew.% eines oder mehrerer Tenside.

2. Verwendung von Magnesiumchlorid in einer Menge von 4-30, vorzugsweise 10-25 Gew.-% (berechnet als MgCl₂ . 6H₂O) gemäß Anspruch 1.

3. Konservierungsmittelfreies, selbst konservierendes, flüssiges, wasserhaltiges Tensidsystem in Form eines Körperreinigungsmittels oder Handgeschirrspülmittels enthaltend 2-50 Gew.-%, vorzugsweise 5-35 Gew.-% einer Tensidkombination, dadurch gekennzeichnet, daß die Tensidkombination aus
a) 20-90 Gew.-%, vorzugsweise 67-75 Gew.-% eines Sulfobernsteinsäure-Halbesters der allgemeinen Formel 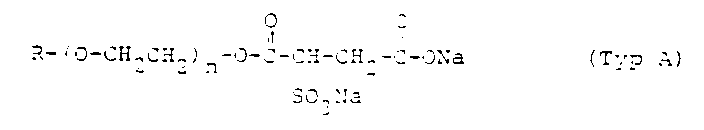 worin R C₇-C₁₇-Alkyl oder Rizinol-Alkyl und n = 2 - 4 bedeuten.
b) 9-79, vorzugsweise 9-49 Gew.-%, insbesondere 22-30 Gew.-% einer oder mehrerer Verbindungen aus der Gruppe der anionischen Tenside.
c) 1-10 Gew.-%, vorzugsweise 2-5 Gew.-% einer oder mehrerer amphoterer Verbindungen
d) 0-5 Gew.-%, vorzugsweise 1-2 Gew.-% einer oder mehrerer nichtionischen Tenside besteht und weiterhin dadurch gekennzeichnet,
daß das System 2-25 Gew.-%, vorzugsweise 5-15 Gew.-% (berechnet als wasserfreies Salz) Magnesiumchlorid enthält.

4. Tensidsystem gemäß Anspruch 3, dadurch gekennzeichnet, daß das Magnesiumchlorid in einer Menge von 4-30, vorzugsweise 10-25 Gew.-% (berechnet als MgCl₂ . 6H₂O) eingesetzt wird.

5. Tensidsystem gemäß einem der Ansprüche 3 oder 4, dadurch gekennzeichnet, daß als Sulfobernsteinsäureester (a) eine Kombination der Sulfobernsteinsäure-Halbester vom Typ A 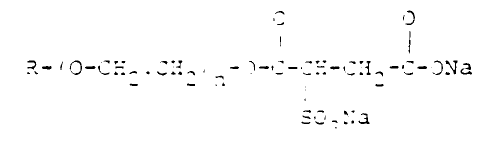 mit Derivaten des
Typs B 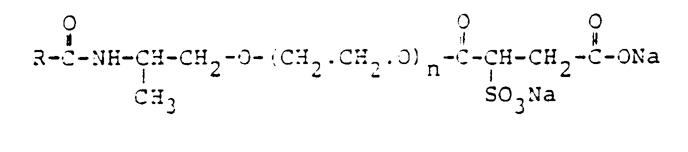 und Typs C 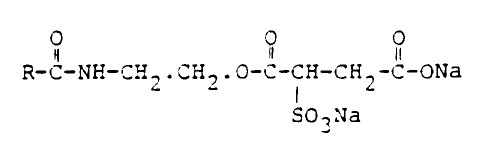 in einem Verhältnis von A:B:C von (2-15):(1-2):1, vorzugsweise (2-10):1:1 eingesetzt wird.

6. Tensidsystem nach einem der Ansprüche 3-5, dadurch gekennzeichnet, daß als anionisches Tensid (b) ein Alkylsulfat. Alkyläthersulfat, sek. Alkansulfonat und/oder Olefinsulfonat verwendet wird.

7. Tensidsystem nach einem der Ansprüche 3-6, dadurch gekennzeichnet, daß als amphotere Verbindung (c) ein (Amido-)Alkylbetain der allgemeinen Formel 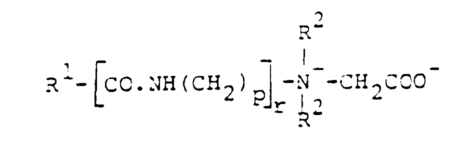 worin R¹ C₇-C₁₇-Alkyl, R² C₁-C₄-Alkyl,
p = 2 oder 3 und r = 0 oder 1 bedeuten, verwendet wird.

8. Tensidsystem nach einem der Ansprüche 3-7, dadurch gekennzeichnet, daß als nichtionisches Tensid (d) ein Fettsäureglycerinpolyethylenglycolester, Fettsäurepolyethylenglycolester und/oder Glycerinethoxylat verwendetwird.

9. Tensidsystem nach einem der Ansprüche 3-8 in Form eines Duschgels.

10. Tensidsystem nach Anspruch 9 in Form eines Duschgels bestehend aus 12-30 Gew.-% einer Tensidkombination (T) aus
58-62 Gew.-% Di-Na-Laurylalkoholpolyglycolethersulfosuccinat,
6-8 Gew.-% Di-Na-Cocosfettsäureisopropanolamidpolyglycolethersulfosuccinat,
5-7 Gew.-% Di-Na-Rizinolsäuremonoethanolamidsulfosuccinat,
22-25 Gew.-% Na-Laurylethersulfat (2-3 Mol EO),
2-4 Gew.-% Cocosfettsäure-amidopropylbetain.
0,5-1,5 Gew.-% Glycerin-Polyethylenglycoloxystearat;
0,5-2,0 Gew.-% 1,2-Propylenglycol,
0,4-1,5 Gew.-% Parfümöl,
und
2-14 Gew.-% Magnesiumchlorid (wasserfrei)
Rest Wasser.

## Claims

1. Use of magnesium chloride in a quantity of at least 2 wt.%, preferably at least 5 wt.% (calculated as anhydrous salt), to preserve preservative-free, liquid, aqueous surfactant systems in the form of a body cleaning agent or manual dishwashing detergent containing 2-50 wt.%, preferably 5-35 wt.% of one or more surfactants.

2. Use of magnesium chloride in a quantity of 4-30, preferably 10-25 wt.% (calculated as MgCl₂·6H₂O) according to claim 1.

3. Preservative-free, self-preserving, liquid, aqueous surfactant system in the form of a body cleaning agent or manual dishwashing detergent containing 2-50 wt.%, preferably 5-35 wt.% of a surfactant combination, characterised in that the surfactant combination consists of
a) 20-90 wt.%, preferably 67-75 wt.% of a sulphosuccinic acid semi-ester of the general formula 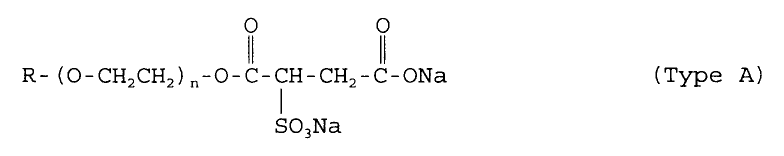 in which R means C₇-C₁₇ alkyl or ricinoleic-alkyl and n = 2-4,
b) 9-79, preferably 9-49 wt.%, in particular 22-30 wt.% of one or more compounds from the group of anionic surfactants,
c) 1-10 wt.%, preferably 2-5 wt.% of one or more amphoteric compounds,
d) 0-5 wt.%, preferably 1-2 wt.% of one or more nonionic surfactants
and further characterised in that the system contains 2-25 wt.%, preferably 5-15 wt.% (calculated as anhydrous salt) of magnesium chloride.

4. Surfactant system according to claim 3, characterised in that the magnesium chloride is used in a quantity of 4-30, preferably 10-25 wt.% (calculated as MgCl₂·6H₂O).

5. Surfactant system according to one of claims 3 or 4, characterised in that the sulphosuccinic acid semi-ester (a) used is a combination of sulphosuccinic acid semi-esters of type A 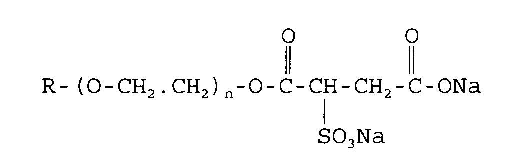 with derivatives of
type B 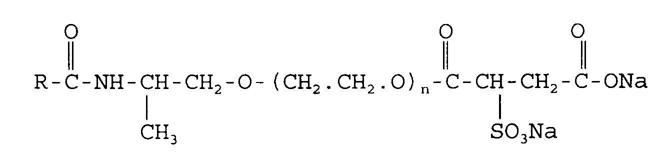 and type C 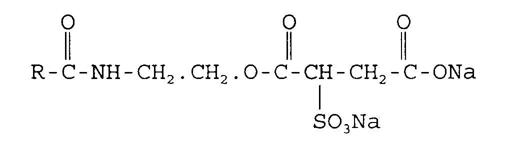 in a ratio of A:B:C of (2-15):(1-2):1, preferably (2-10):1:1.

6. Surfactant system according to one of claims 3-5, characterised in that the anionic surfactant (b) used is an alkyl sulphate, alkyl ether sulphate, sec. alkane sulphonate and/or olefin sulphonate.

7. Surfactant system according to one of claims 3-6, characterised in that the amphoteric compound (c) used is an (amido)alkylbetaine of the general formula 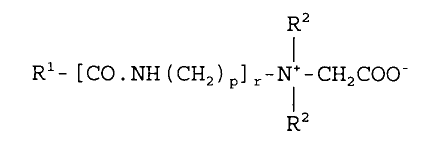 in which R¹ means C₇-C₁₇ alkyl, R² means C₁-C₄ alkyl,
p = 2 or 3 and r = 0 or 1.

8. Surfactant system according to one of claims 3-7, characterised in that the nonionic surfactant (d) used is a fatty acid glycerol polyethylene glycol ester, fatty acid polyethylene glycol ester and/or glycerol ethoxylate.

9. Surfactant system according to one of claims 3-8 in the form of a shower gel.

10. Surfactant system according to claim 9 in the form of a shower gel consisting of 12-30 wt.% of a surfactant combination (T) prepared from
58-62 wt.% di-Na lauryl alcohol polyglycol ether sulphosuccinate,
6-8 wt.% di-Na coconut fatty acid isopropanolamide polyglycol ether sulphosuccinate,
5-7 wt.% di-Na ricinoleic acid monoethanolamide sulphosuccinate,
22-25 wt.% Na lauryl ether sulphate (2-3 mol EO),
2-4 wt.% coconut fatty acid amidopropyl betaine,
0.5-1.5 wt.% glycerol polyethylene glycol oxystearate;
0.5-2.0 wt.% 1,2-propylene glycol,
0.4-1.5 wt.% perfume oil,
and
2-14 wt.% magnesium chloride (anhydrous)
remainder water.

## Revendications

1. Utilisation de chlorure de magnésium dans une proportion d'au moins 2% en poids, de préférence d'au moins 5% en poids (calculée sur la base du sel anhydre), pour la conservation de systèmes tensio-actifs aqueux liquides, exempts d'agents conservateurs, sous forme d'un produit de nettoyage corporel ou d'un produit de lavage de la vaisselle à la main, contenant 2 à 50% en poids, de préférence 5 à 35% en poids d'un ou de plusieurs tensio-actifs.

2. Utilisation de chlorure de magnésium dans une proportion de 4 à 30%, de préférence 10 à 25% en poids (calculée sur la base de MgCl₂ 6N₂O) selon la revendication 1.

3. Système tensio-actif aqueux liquide, autoconservateur et exempt d'agents conservateurs, contenant 2 à 50% en poids, de préférence 5 à 35% en poids d'une combinaison de tensio-actifs, caractérisé en ce que la combinaison de tensio-actifs se compose de
a) 20-90% en poids, de préférence 67-75% en poids d'un semi-ester d'acide sulfosuccinique de formule générale 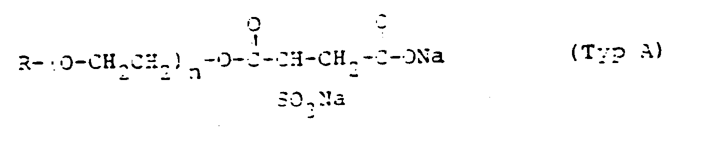 dans laquelle R représente un reste alkyle en C₇-C₁₇ ou ricinolakyle et n = 2-4
b) 9-79, de préférence 9-49% en poids, en particulier 22-30% en poids d'un ou de plusieurs composés du groupe des tensio-actifs anioniques,
c) 1-10% en poids, de préférence 2-5% en poids d'un ou de plusieurs composés amphotères
d) 0-5% en poids, de préférence 1-2 % en poids d'un ou de plusieurs tensio-actifs non ioniques,
et caractérisé en outre en ce que le système contient 2-25 % en poids, de préférence 5-15% en poids (calculés sur la base du sel anhydre) de chlorure de magnésium.

4. Système tensio-actif selon la revendication 3, caractérisé en ce qu'on utilise du chlorure de magnésium dans une proportion de 4 à 30, de préférence de 10 à 25% en poids (calculée sur la base de MgCl₂ 6H₂O).

5. Système tensio-actif selon l'une ou l'autre des revendications 3 et 4, caractérisé en ce qu'on utilise, comme ester d'acide sulfosuccinique (a), une combinaison du semi-ester d'acide sulfosuccinique du type A 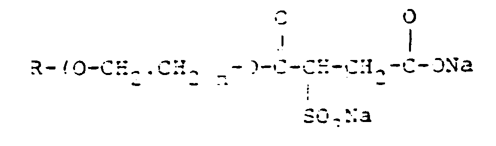 avec des dérivés du type B 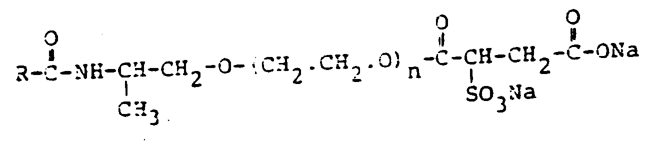 et du type C 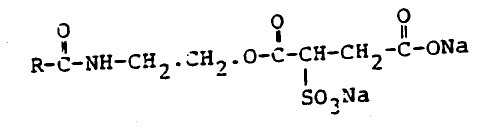 dans un rapport A:B:C de (2-15):(1-2):1, de préférence de (2-10):1:1

6. Système tensio-actif selon l'une quelconque des revendications 3 à 5, caractérisé en ce qu'on utilise, comme tensio-actif anionique (b), un alkylsulfate, un alkyléthersulfate, un alcane secondaire sulfonate et/ou un oléfine sulfonate.

7. Système tensio-actif selon l'une quelconque des revendications 3 à 6, caractérisé en ce qu'on utilise, comme composé amphotère (c), une (amino-)alkylbétaïne de formule générale 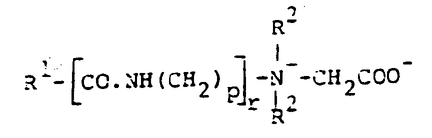 dans laquelle R¹ représente un reste alkyle en C₇-C₁₇, R² un reste alkyle en C₁-C₄, p = 2 ou 3 et r = 0 ou 1.

8. Système tensio-actif selon l'une quelconque des revendications 3 à 7, caractérisé en ce qu'on utilise, comme tensio-actif non ionique (d), un ester de polyéthylèneglycol, de glycérol et d'acide gras, un ester de polyéthylèneglycol et d'acide gras et/ou un éthoxylate de glycérol.

9. Système tensio-actif selon l'une quelconque des revendications 3 à 8, sous la forme d'un gel de douche.

10. Système tensio-actif selon la revendication 9 sous la forme d'un gel de douche, se composant de 12 à 30% en poids d'une combinaison de tensio-actifs (T) constituée par
58-62% en poids de sulfosuccinate d'oxyde de polyglycol et d'alcool laurique disodé,
6-8% en poids de sulfosuccinate d'oxyde de polyglycol, d'isopropanolamide et d'acide d'huile de coco disodé,
5-7% en poids de sulfosuccinate de monoéthanolamide et d'acide ricinoléique disodé.
22-25% en poids de lauryléthersulfate sodé (2-3 moles d'EO),
2-4% en poids d'amidopropylbétaïne d'acide d'huile de coco,
0,5-1,5% en poids d'oxystéarate de polyéthylèneglycol et de glycérol ; et de
0,5-2,0% en poids de 1,2-propylèneglycol,
0,4-1,5% en poids de parfum et
2-14% en poids de chlorure de magnésium (anhydre),
le reste étant de l'eau.
